# EUROPEAN PATENT APPLICATION

(11) **EP 1 445 711 A2**
(43) Date of publication of application: **11.08.2004**
(21) Application number: 04000620.7
(22) Date of filing: 14.01.2004
(51) Int. Cl.: G06F 17/30

(54) **Systems and methods for constructing and using models of memorability in computing and communications applications**

(30) Priority: 04.02.2003 US 444827 P; 25.02.2003 US 374436
(71) Applicant: MICROSOFT CORPORATION, Redmond, Washington 98052 (US)
(72) Inventor: Horvitz, Eric J., Kirkland Washington 98003 (US); Dumais, Susan T., NE Kirkland Washington 98033 (US); Ringel, Meredith J., Stanford California 94305 (US); Cutrell, Edward B., Seattle Washington 98103 (US); Koch, Paul B., East Seattle Washington 98112 (US)
(74) Representative: Grünecker, Kinkeldey, Stockmair & Schwanhäusser Anwaltssozietät

(57) **Abstract**

One or more models of memorability are provided that facilitate various computer-based applications including those centering on the storage, retrieval, and processing of information, applications that remind people about items they nsk not recalling or overlooking, and facilitating communications of reminders. In one application, the models are used to help compose and navigate large personal stores of information about a user's activities, communications, images, and other content. In another application, views of files in directories are extended with the addition of memory landmarks, and a means for controlling the number of landmarks provided *via* changing a threshold on inferred memorability. Another application centers on the use of models of memorability to select subsets of images from larger sets representing events, for display in a slide show or ambient photo display. In another application, a system is provided that facilitates computer-based searching for information by providing for the design and analysis of timeline visualizations in connection with displaying results to queries based at least in part on an index of content. A query is received by a query component (which can be part of search engine that provides a unified index of information a user has been exposed to). The query component parses the query into portions relevant to effecting a meaningful search in accordance with the subject invention. The query component can access and populate a data store which may include information searched for. A landmark component receives and/or accesses information from the query component as well as the data store, and anchors public and/or personal landmark events to search results-related information.

## Description

### REFERENCE TO RELATED APPLICATION(S)

This application claims the benefit of U.S. Provisional Patent Application Serial No. 60/444,827 which was filed February 04, 2003, entitled System and Method That Facilitates Computer-Based Searching For Content, the entirety of which is incorporated herein by reference.

### TECHNICAL FIELD

This invention is related to systems and methods that facilitate computer-based applications in accordance with one or more memorability models that capture the ability of people to recognize particular events as important landmarks in time and to benefit by using the landmarks in navigating or reviewing content.

### BACKGROUND OF THE INVENTION

Global competition has led to an ever-increasing demand for accessing relevant information quickly. For example, prompt access to relevant information can make a difference with respect to making money over losing money in the stock market. Demands on the media and journalists place a premium on obtaining relevant information before the competition. Other industries such as in the high technology sector and consulting fields require individuals in those industries to be on top of current events and trends with respect to certain markets. Likewise, within a client-based system and intranet, quickly accessing relevant information is a must with respect to remaining efficient within a working environment. Accordingly, there is an ever-increasing need for systems and methods which facilitate prompt access to relevant information.

### SUMMARY OF THE INVENTION

The following presents a simplified summary of the invention in order to provide a basic understanding of some aspects of the invention. This summary is not an extensive overview of the invention. It is not intended to identify key/critical elements of the invention or to delineate the scope of the invention. Its sole purpose is to present some concepts of the invention in a simplified form as a prelude to the more detailed description that is presented later.

The present invention provides systems and methods for developing and harnessing models of memorability that capture in an automated manner the ability of people to recognize events as important landmarks in time. The models of memorability include procedures and policies for categorizing or assigning some measure of memorability to events that can be employed by various computer-based applications to aid users in processing, receiving, and/or communicating information. As an example, events can include appointments and other annotations in a user's calendar, holidays, news stories over time, and photos, among other items. In one particular example application, the models are employed to provide a personalized index containing landmarks in time, wherein the use of such an index can be utilized in browsing directories of files or other information and in reviewing the results of a search engine. The memorability models can include voting models, heuristic models, rules models, statistical models, and/or complimentary models that are based on patterns of forgetfulness rather then items remembered. In addition, user interfaces are provided that facilitate application of the models to assisting users in the retrieval and processing of information. Furthermore, the present invention includes various applications and methods for building a data store itself such as providing a browsable archive of important (and less important) data. For example, the data store can capture a life history (or other events) such as "Our families biography," and "My autobiography" and so forth.

In another aspect, the subject invention provides for a system and method that facilitates computer-based searching for information in accordance with the memorability models. This includes design and analysis of timeline visualizations in connection with displaying results to queries based at least in part on an index of content. The visualization in connection with the subject invention can be related to a search engine that provides a unified index of information a user has been exposed to (*e.g*., including web pages, email, documents, pictures, audio...). The subject invention exploits value of extending a basic time view by adding public landmarks (*e.g*., holidays, important news events) and/or personal landmarks (*e.g*., photos, significant calendar events). According to one particular aspect of the invention, results of searches can be presented with an overview-plus-detail timeline visualization. A summary view can show distribution of search hits over time, and a detailed view allows for inspection of individual search results. Returned items can be annotated with icons and short descriptions, if desired.

People employ a variety of strategies when searching through personal emails, files, or web bookmarks for a particular item. Although people do not remember all aspects of an item they are looking for (such as for example an exact title and path of a file), they do tend to remember important events in their lives (*e.g*., their children's birthdays, exotic travel, prominent events such as the 9/11 attacks or the assassination of JFK). The subject invention can employ such types of contextual information to support searching through content. Interactive visualization in accordance with the subject invention provides timeline-based presentations of search results that can be anchored by public (*e.g*., news, holidays) and/or personal (*e.g*., appointments, photos) landmark events. An indexing and search system underlying the visualization in accordance with the subject invention can index text and metadata of items (*e.g*., documents, visited web pages, and emails) that a user has been exposed to so as to provide a fast and easy manner to search over and retrieve information content.

To the accomplishment of the foregoing and related ends, certain illustrative aspects of the invention are described herein in connection with the following description and the annexed drawings. These aspects are indicative, however, of but a few of the various ways in which the principles of the invention may be employed and the present invention is intended to include all such aspects and their equivalents. Other advantages and novel features of the invention may become apparent from the following detailed description of the invention when considered in conjunction with the drawings.

### BRIEF DESCRIPTION OF THE DRAWINGS

Fig. 1 is a high-level schematic illustration of various memorability models that can be employed with computer-based applications in accordance with an aspect of the present invention.
Figs. 2-5 illustrate exemplary user interfaces in accordance with an aspect of the present invention.
Figs. 6 and 7 illustrate exemplary influence models in accordance with an aspect of the present invention.
Figs. 8 and 9 illustrate exemplary decision trees in accordance with an aspect of the present invention.
Fig. 10 illustrates exemplary display controls in accordance with an aspect of the present invention.
Fig. 11 is a high-level schematic illustration of an exemplary system in accordance with the subject invention.
Fig. 12 is a flow diagram of one particular methodology in accordance with the subject invention.
Fig. 13 is an exemplary screenshot representation of a timeline visualization in accordance with the subject invention.
Fig. 14 is a representative visualization displaying only dates to the left of a timeline's backbone.
Fig. 15 is a representative visualization displaying landmarks (e.g., holidays, news headlines, calendar appointments, and personal photographs) in addition to basic dates.
Fig. 16 illustrates that median search time with landmark events displayed in a timeline in accordance with the subject invention was significantly faster than median search time when only dates were used to annotate the timeline.
Fig. 17 is an exemplary operating environment in accordance with the subject invention.

### DETAILED DESCRIPTION OF THE INVENTION

The present invention is now described with reference to the drawings, wherein like reference numerals are used to refer to like elements throughout. In the following description, for purposes of explanation, numerous specific details are set forth in order to provide a thorough understanding of the present invention. It may be evident, however, that the present invention may be practiced without these specific details. In other instances, well-known structures and devices are shown in block diagram form in order to facilitate describing the present invention.

As used in this application, the terms "component," "system," "model," "application," and the like are intended to refer to a computer-related entity, either hardware, a combination of hardware and software, software, or software in execution. For example, a component may be, but is not limited to being, a process running on a processor, a processor, an object, an executable, a thread of execution, a program, and/or a computer. By way of illustration, both an application running on a server and the server can be a component. One or more components may reside within a process and/or thread of execution and a component may be localized on one computer and/or distributed between two or more computers.

As used herein, the term "inference" refers generally to the process of reasoning about or inferring states of the system, environment, and/or user from a set of observations as captured via events and/or data. Inference can be employed to identify a specific context or action, or can generate a probability distribution over states, for example. The inference can be probabilistic - that is, the computation of a probability distribution over states of interest based on a consideration of data and events. Inference can also refer to techniques employed for composing higher-level events from a set of events and/or data. Such inference results in the construction of new events or actions from a set of observed events and/or stored event data, whether or not the events are correlated in close temporal proximity, and whether the events and data come from one or several event and data sources.

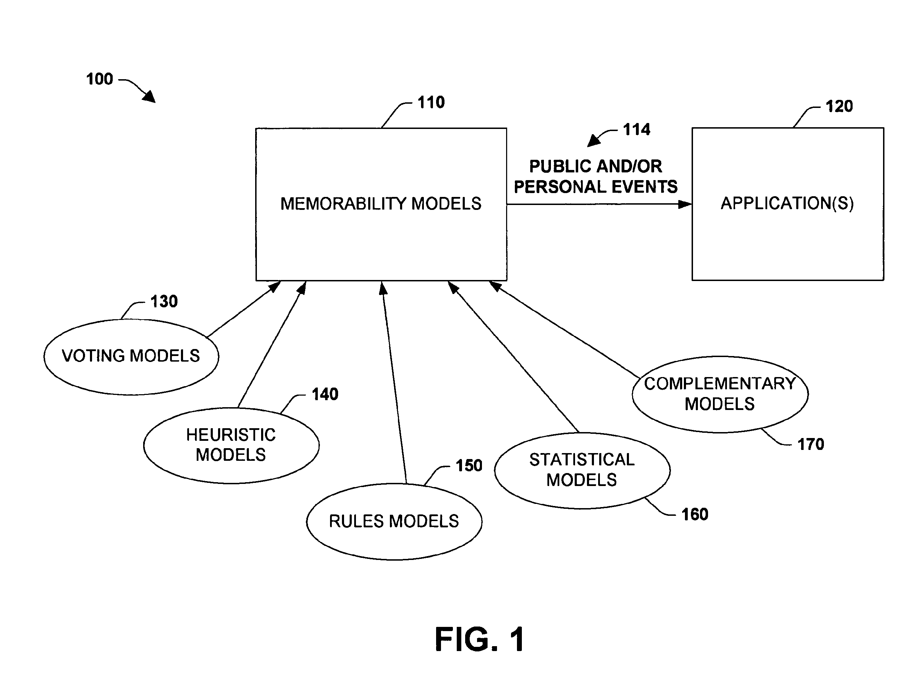

Referring initially to Fig. 1, a system 100 illustrates one or more memorability models that can be employed with computer-based applications in accordance with an aspect of the present invention. One or more memorability models 110 are provided that drive one or more applications 120 that aid users in the management, retrieval, processing and/or communications of information. The memorability models 110 determine various aspects of people or users remembrance of one or more events 114 (*e.g.*, public and/or private memories), and in some cases, the models can be based upon forgetfulness rather than an ability to recall. As can be appreciated, remembrance and forgetfulness models can be employed concurrently in accordance with the present invention. In one aspect, the memorability models 110 can employ a shared voting model 130 to determine memorable items. For example, this can include asking or automatically polling a set of users to score the memorability of public events. In one example, scalar measures of memorability can be collected that may include salience of news stories taken from a corpus of news stories, by querying a set of people to assign a value of 1-10 (or other scoring system), thus, capturing how memorable a news story is by averaging the scores (or other statistical process).

One or more heuristic models 140 can be provided as a memorability model 110. For example, these models 140 can utilize several properties of messages and create informal policies that assign scores or deterministic categories of memorability based on functions of properties. As an example, a heuristic function can be constructed that analyzes the increasing duration of events on a calendar (or other information source) as positively influencing the memorability of events. This can include considerations of heuristics relating to which images or subsets of images from a set of images would serve as the most memorable of sets of images snapped at an event, based on such properties as the pictures themselves, including composition of objects in a scene, color histogram, faces recognized (*e.g.*, by automated face recognition software), features involving the sequence and temporal relationships among pictures (*e.g*., first, or near first in a set of pictures snapped to capture an event), a picture associated with short inter-picture intervals, capturing excitement of the photographer about an aspect of the event 114, and properties that indicate that a user's activity with regard to the picture, such as having examined or displayed (with relatively longer dwell time on the picture) the image, having edited (*e.g*., cropped and renamed) the picture, and so forth. Other features of images include automated analysis of image quality, including focus and orientation, for example.

At 150, one or more rules models or rules can be provided to determine events 114. This can include rules for automatically assigning measures of memorability to news stories that can include such properties as the number of news stories, persistence in the media, number of casualties, the dollar value of the loss associated with the news story, features capturing dimensions of surprise or atypical, and the proximity to the user of the event (*e.g*., same/different country, state, city, and so forth). At 160, various statistical models can be provided to model the events 114. Statistical models 160 may be employed for various items, centering on the use of machine learning methods that can provide models which can predict the memorability of items, including calendar events, holidays, news stories, and images, based on sets of features, and so forth. Statistical models 160 and process include the use of Bayesian learning, which can generate Bayesian dependency models, such as Bayesian networks, naïve Bayesian classifiers, and/or Support Vector Machines (SVMs), for example. A trainer (not shown) can be supplied that takes explicit examples of landmark items―or items that may be most likely forgotten, depending on the application, or can be supplied with examples identified through implicit training.

Models of memorability 110 can be also be formulated in a complementary manner at 170 to yield models of forgetting, and thus can be leveraged in the applications 120. Thus, the complimentary models 170 describe the use of variants of the models of memorability 110 which are focused on inferring the likelihood that users *will not* recall an important forthcoming event or other related information. These models 170 can utilize inferences in applications 120, such as calendars to highlight in a selective manner the information that a user is likely to forget in a visually salient manner, or to change the timing or alerting of information in accordance with the likelihood that the information will not be remembered. Such models of memorability and forgetting can be combined with messaging and reminding systems, for example, wherein context-sensitive costs and benefits of transmitting the information and alerting a user, about information that they may need because they will not remember it, (*e.g*., information transmitted to a peripheral device or display can be considered in an informal cost-benefit analysis or a formal decision analysis that consider the expected value of if, when, and how to step forward with a reminder). As will be described in more detail below, views of events over time, and processes for assisting users can be provided to browse information stores, in the context of sets of events that are important for easing the task of identifying documents created over time.

The memorability models 110 support various systems, processes, and applications 120. This can include employing model of memorability information-management applications that labels events or items with numerical or categorical labels according to some measure of the likelihood that an item will be recalled, recognized as a landmark, or be most representative of an event or time. These applications can utilize mathematical functions that assign a scalar measure of salience of events or items as being recalled, recognized as landmarks, or be most representative of events or times. Statistical models of memorability via machine learning methods can also be applied, trained implicitly or with an explicit training system that collects information about a sample of memorable or non-memorable events or items. This can include providing real-time inference or classification about the likelihood that events or items as being recalled, recognized as landmarks, or be most representative of events or times, or, more generally, provide a probability distribution over different degrees or aspects of the systems and processes supported by the present invention.

Other applications include the use of models of memorability to automatically filter a stream of heterogeneous events and content, so as to selectively store events for log of lifetime events, for example, to limit required memory of storage. The use of models of memorability can also be employed to create a means of browsing (*e.g*., hierarchically a lifetime log of heterogeneous events or content browsing data at different levels of temporal precision (*e.g*., hours, days, months, years, decades)). Another application includes the use of models of representative landmarks and memorability to selectively choose pictures for an ambient display of pictures drawn from a picture library. Still other applications include the use of models of representative memory landmarks and memorability to selectively choose a set of pictures in a slide show over time or at different points in time about one or more events, under constraints in the total number of slides that a user desires to show. In yet another aspect, applications include the use of models of representative memory landmarks and memorability to selectively choose a set of items (*e.g*., images) to characterize or summarize the contents of a corpus of items (*e.g*., a photo library, thumbnails of graphics or photo images displayed on the files, items, or folders of documents in an operating system (e.g., MS Windows). It is noted that the concepts of memorability also apply to a range of targets, per learning and inference such as:
**Memorability**: The degree to which an item will be recalled or recognized.
**Memorable landmark**: The degree to which an item will be viewed as a milestone in time, useful for navigation and indexing.
**Representative landmark**: The degree to which an item serves as a representative for items, a period of time, events, sequence of events, etc.

As noted above, a complement to models of memorability are models of forgetting. Thus, the present invention can similarly train models from data and perform inference about items that may be forgotten and couple the inferred likelihood that an item will be forgotten with a cost-benefit analysis of the expected value of reminding a user about an item. General decision-theoretic analyses about when to come forward under the uncertainty that assistance is needed is described by works such as *Principles of Mixed-Initiative Interaction* by E. Horvitz, Proceedings of CHI'99, ACM SIGCHI Conference on Human Factors in Computing Systems, Pittsburgh, PA, May 1999. ACM Press. pp 159-166.

The present invention can employ such expected-utility methods, taking as central in the computation of the expected value of reminding a user, the likelihood of forgetting (and remembering) that is inferred from models of memorability. Thus, the present invention can perform expected-utility decision making about if and when to come forward to remind a user about something that they are likely to forget given the item type and context―considering the cost of the interruption (*e.g*., the current cost of interruption). Such models can be used in the control of alerting about reminders in desktop, as well for mobile devices, via the incorporation of the disruptiveness and the cost of the transmission.

Beyond use for healthy people, such models can also be exploited to assist patients with various cognitive deficits that may lead to memory aberrancies. For example, a model of memorability built from training data may be used to predict the likelihood that a patient with Alzheimer's disease is at a particular stage of the illness. Such models can be coupled with cost-benefit analyses as described above and, with appropriate hardware to provide audiovisual cues to users, providing ideal reminders.

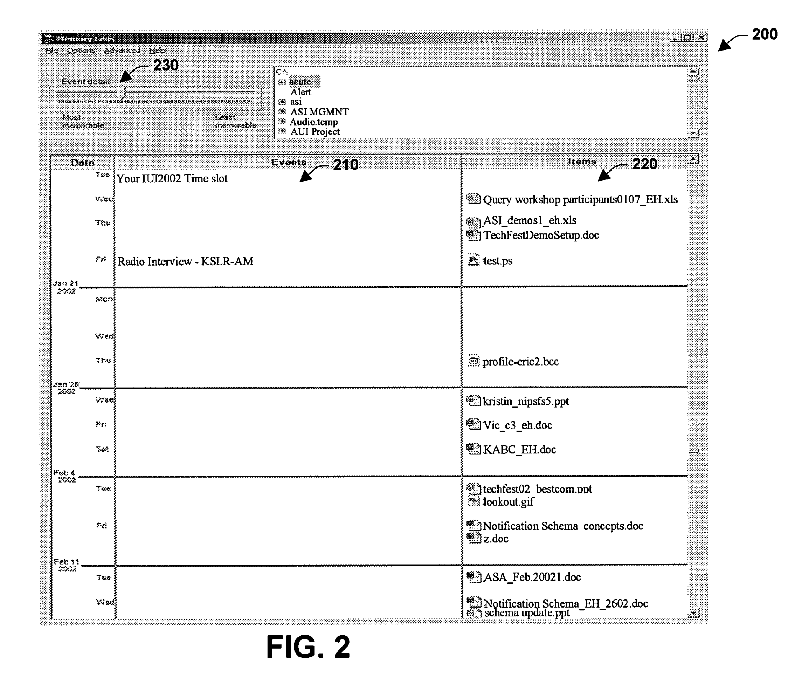

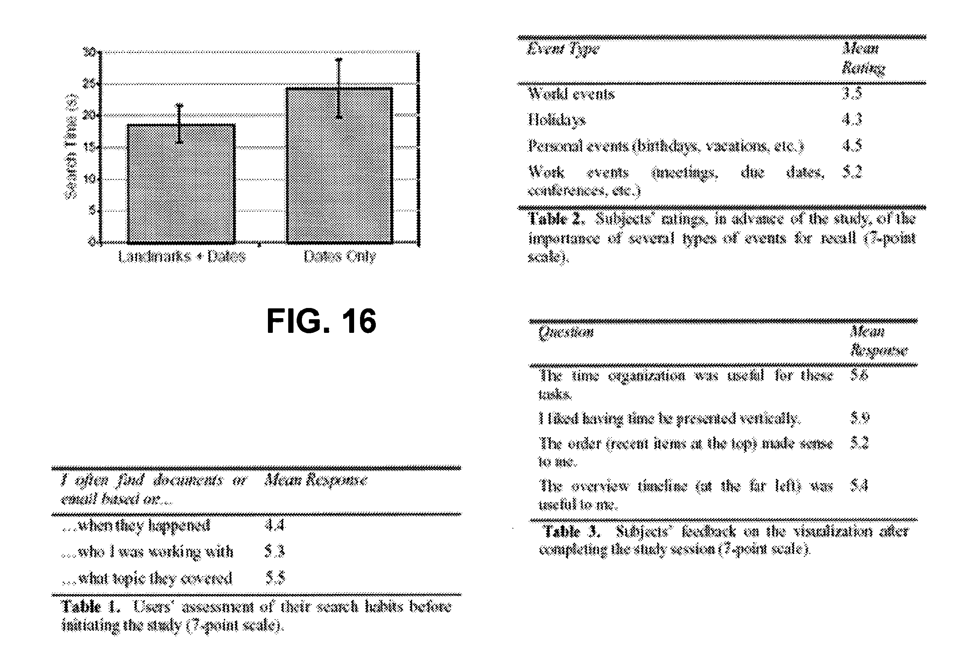

Figs. 2-17 illustrate some example interfaces that utilize memorability models in accordance with the present invention. It is noted that the respective interface depicted can be provided in various other different settings and context. As an example, the applications and/or memorabilty models discussed above can be associated with a desktop development tool, mail application, calendar application, and/or web browser although other type applications can be utilized. These applications can be associated with a Graphical User Interface (GUI), wherein the GUI provides a display having one or more display objects (not shown) including such aspects as configurable icons, buttons, sliders, input boxes, selection options, menus, tabs and so forth having multiple configurable dimensions, shapes, colors, text, data and sounds to facilitate operations with the applications and/or memorability models. In addition, the GUI can also include a plurality of other inputs or controls for adjusting and configuring one or more aspects of the present invention and as will be described in more detail below. This can include receiving user commands from a mouse, keyboard, speech input, web site, remote web service, pattern recognizer, face recognizer, and/or other device such as a camera or video input to affect or modify operations of the GUI.

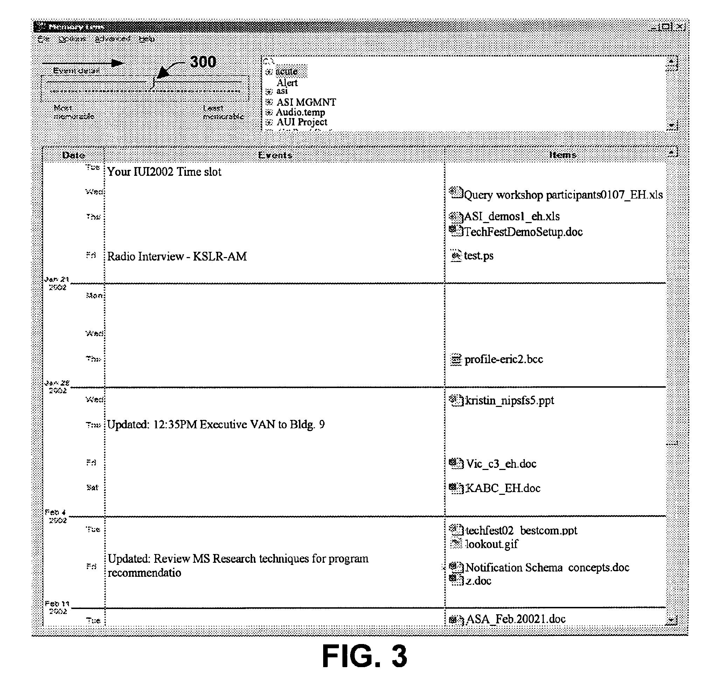

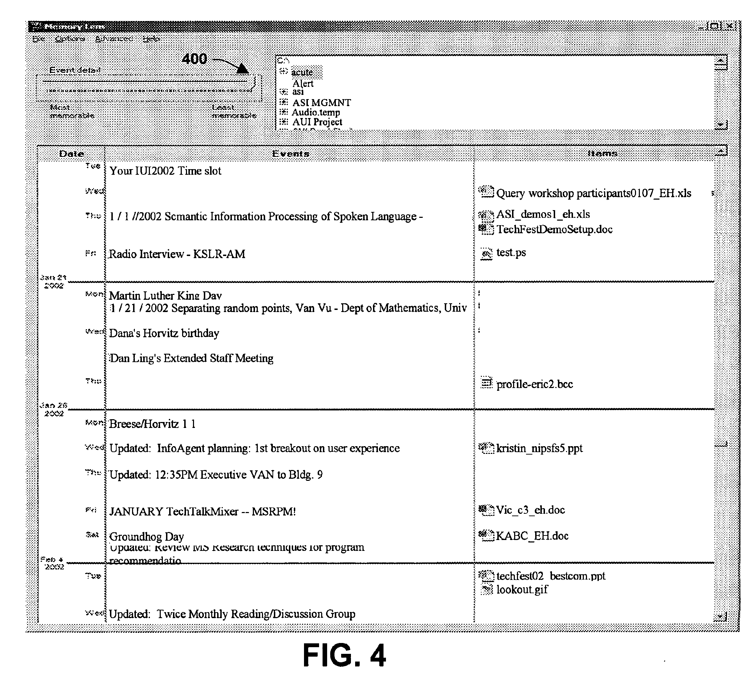

Fig. 2 illustrates an example interface 200 that employs memorability models in accordance with the present invention. The interface 200 (*e.g*., MemoryLens) posts an event backbone on any directory being explored. Important personal events are filtered from all available events and are posted in the left hand column 210. Files or other data created or modified at different times are displayed in the appropriate time period on the right-hand column at 220. A slider 230 is moved towards "most memorable,' landmarks, thus allowing landmark events from a user's calendars to be displayed that have a higher likelihood than a threshold of being memorable, per the setting of the slider 230.
The interface 200 depicts the use of appointment items, however, as can be appreciated it can apply similar methods to adding key images and news stories, etc. to the left hand column 210. Files can be launched directly from these columns (*e.g*., mouse click), as in other file browsers. Fig. 4 illustrates how a slider 300 is moved to the right (in direction of arrow), allowing events to be added of lower probability of being memory landmarks. Thus, more events are added from that depicted in Fig. 3. Proceeding to Fig. 4, a slider 400 is moved further to the right, allowing even more events to be added―that is events of even lower probability of being memory landmarks are now included. As the slider is moved, other events are added, including Ground Hog day, a recurrent meeting with an associate, and a brother's birthday, for example. A display affordance is provided of progressively lightening events with progressively lower likelihoods of being a landmark; in this case, a step function can be introduced that assigns intensity as a function of membership of an event within different ranges of likelihood of being a landmark.

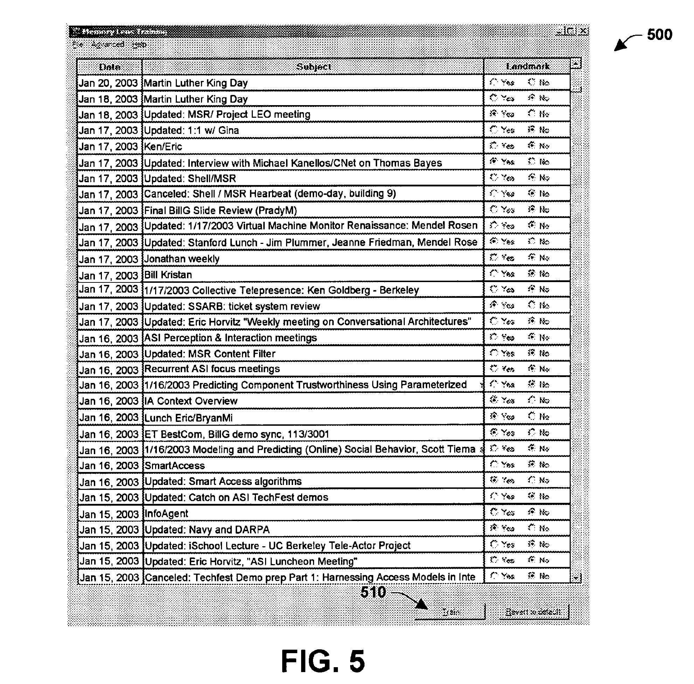

A training system and method can be invoked in the interfaces depicted above. Fig. 5 illustrates an interface 500, wherein a trainer fetches a file of a user's calendar appointments over the years and allows the user to indicate whether appointments serve as memory landmarks or not. The user assigns these labels to some subset of appointments. When the user is finished, he or she hits a "train" button 510, and a statistical classifier is created, that can take multiple properties of events on a user's calendar and predict the likelihood that each event is a landmark event, that is:
p(memory landmark | E1 ...En), wherein p is a probability and E1...En is evidence relating to one or more event properties (*e.g*., closeness of event to holiday, key words such as important or urgent meeting, award presentation or reception indicators, milestone meetings, performance review, and so forth). This probability can be assigned to non-scored calendar events for use in the above interfaces.

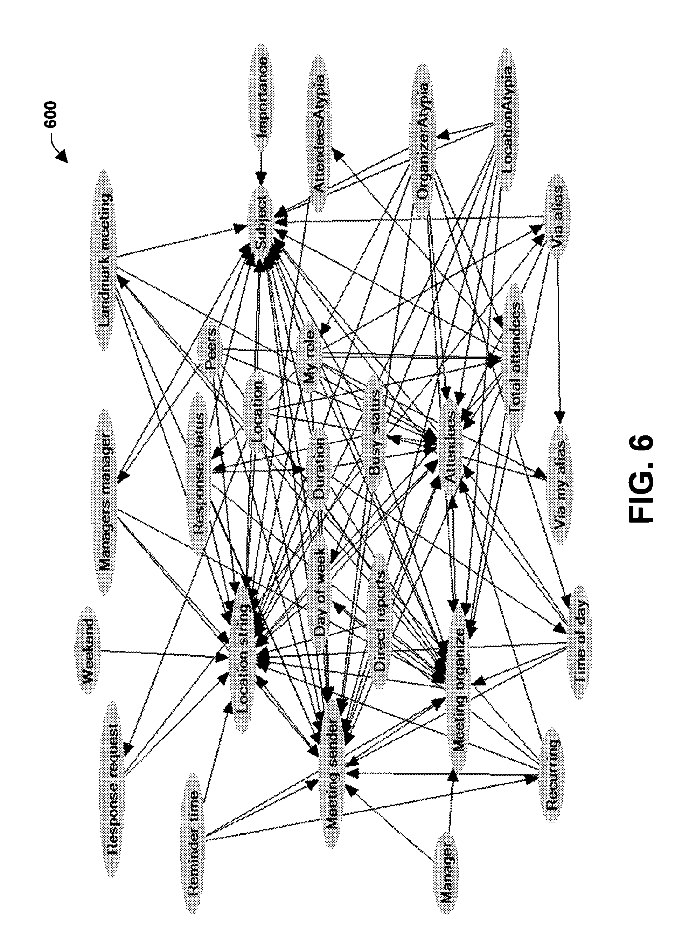

It is noted that one or more decision models can be formulated for computing memorization models. Consider for example, the model 600 displayed in Fig. 6, represented as an influence diagram. Influence diagrams are a well-known representation of decision problems in the decision science community. The models capture uncertain relationships among key variables, including observational variables, decisions, and value functions. The influence diagram, displayed in Fig. 6, captures components that influence memorability from a user's appointments, although other variable sources may be employed. In the model 600, key variables (can include other variables), including observational and inferred variables, are represented by oval nodes in the graph 600. Directed arcs represent probabilistic or deterministic dependence among variables.
The model 600 shows a Bayesian network (probabilistic dependency model) inferred from the data. Note the variables being considered, can be automatically gleaned from a user's online appointments. Some of the more interesting variables include, whether or not peers (organizationally) are at a meeting, the day of week, the time of day, the duration of the meeting, whether the meeting is recurrent, the time set for early reminding about the meeting, the role of the user (organizer?, attendee?, etc.), did the meeting come *via* an alias or from a person, how many attendees are at the meeting, are a user's direct reports, manager, or manager's manager at the meeting, who is the organizer of the meeting, the subject of the meeting, the location of the meeting, how did the user respond to the meeting request. Some variables under consideration (see Bayesian network model) in statistical modeling are specially designed for this kind of memory landmark application. These include "organizer atypia," "location atypia," and "attendees atypia." These are computed from a user's appointment store and capture the rarity or "atypia" of properties of an event or appointment.

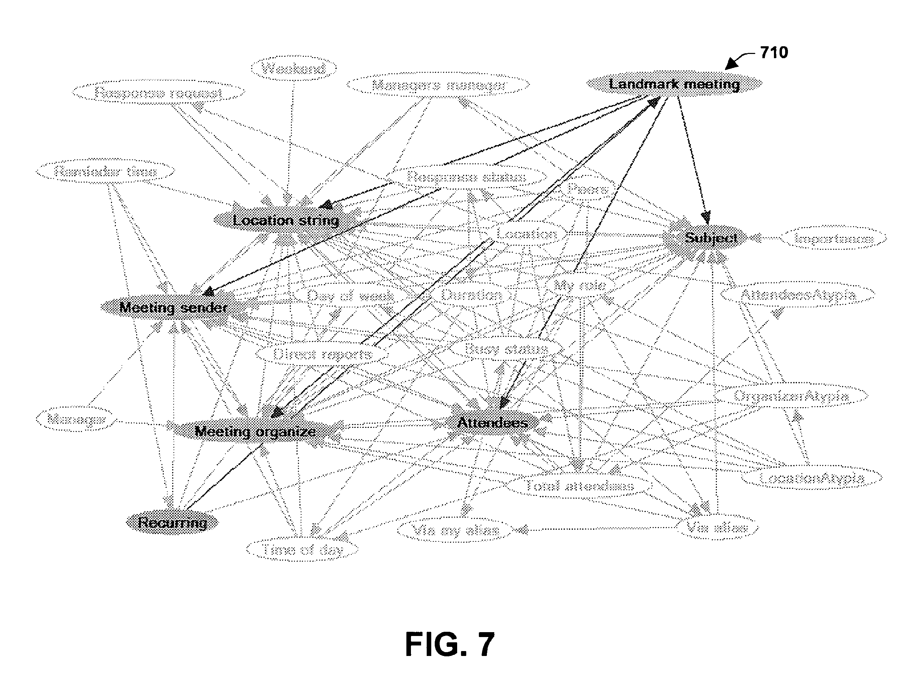

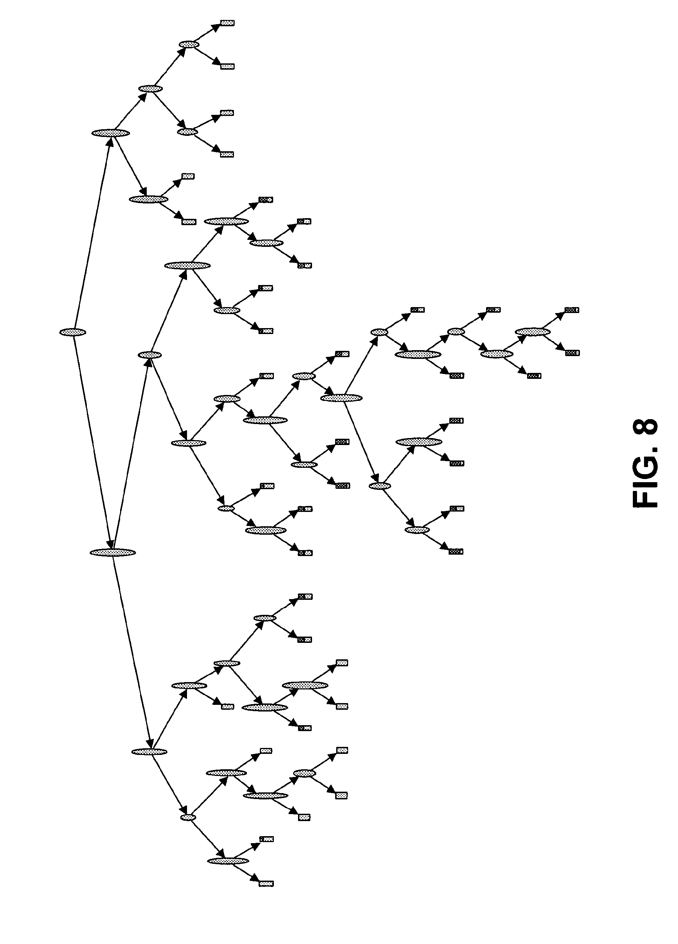

Organizer atypia refers to the frequency that the organizer has organized a meeting. All of the appointments are examined and the organizers are noted. The fraction of times the current organizer has been an organizer for the meetings is computed for each meeting being analyzed. The same is performed for locations and attendees at a meeting. For the latter, the most atypical attendee is considered to be the atypical attendee meeting property for an event. In one implementation, the present invention discretizes typicality for *Location, Organizer,* and *Attendees* into states based on ranges of frequency, *e.g*.,:
0% to 1% - very atypical
1% to 5% - atypical
5% to 10% - typical
10% to 100% - very typicalFig. 7 depicts some of the more important variables from a particular test set―per dependencies directly with a variable representing the likelihood that a meeting is a landmark meeting at 710. Fig. 8 is a decision tree that is generated by a statistical modeling tool. This tree operates inside the "Landmark meeting" variable 710 in Fig. 7.

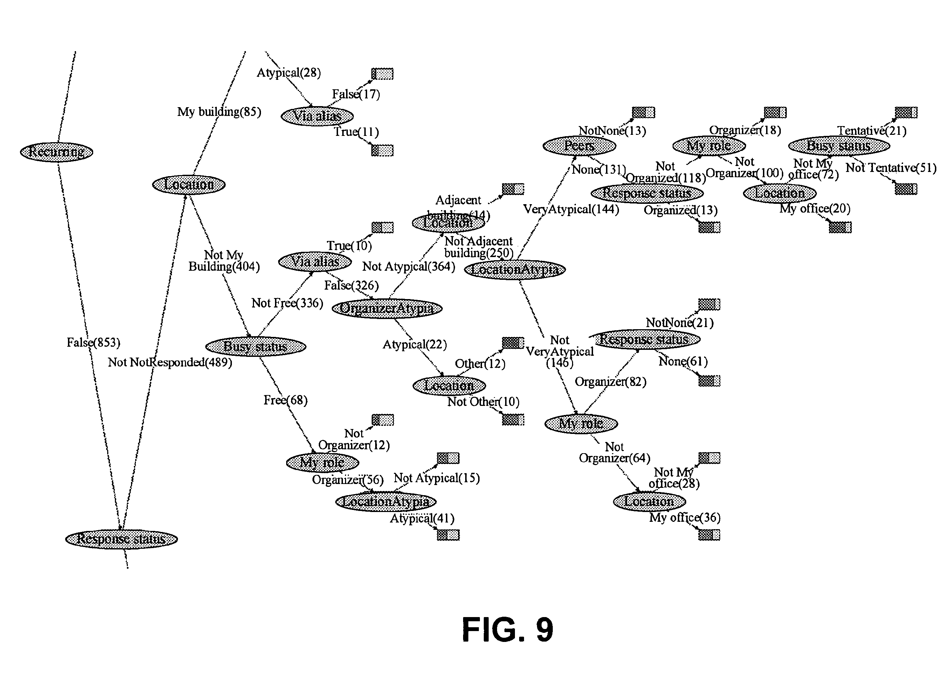

Fig. 9 depicts a zoom in on the middle portion of the decision tree in Fig. 8 for predicting landmark meetings. The length of bars at the leaves of each set of branches or "paths" is the likelihood that a meeting will be considered a landmark meeting. The main branch displayed here represents meetings that are not recurrent, that I have responded to, that are not in my building, and that are marked as busy time. Additional properties are considered in downstream branching.

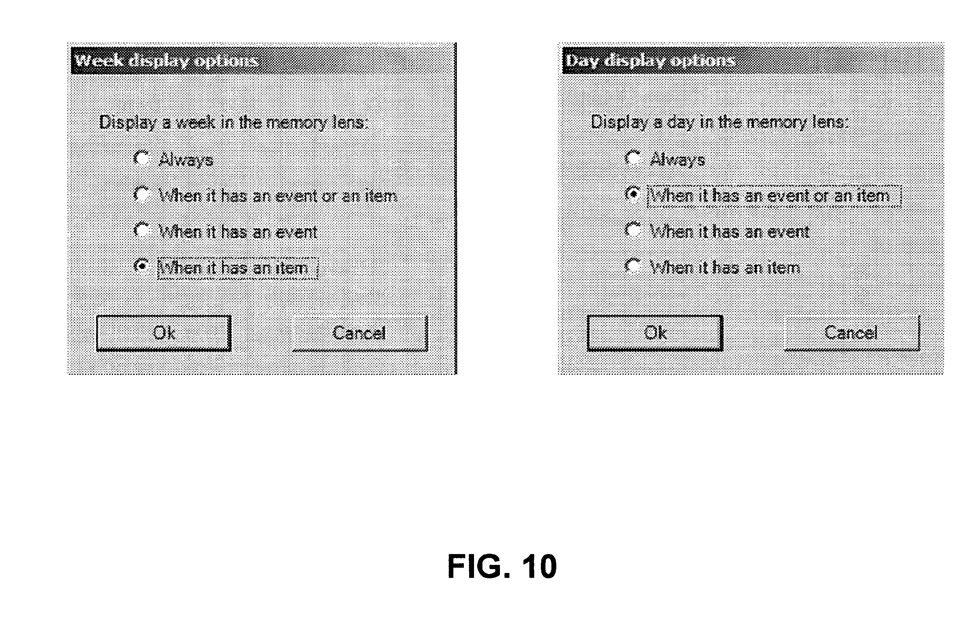

Fig. 10 depicts display controls that may be selected by users for controlling how/when events and items are displayed (*e.g*., always, when it has an event or item, when it has an event, when it has an item). The above interfaces posed some interesting design questions about methods and controls, per preferences for the display of explicit dates and times, based on the existence of documents or other items, and/or events that were above threshold―and for reformatting as more events came above threshold with the movement of the slider thus, controlling the threshold for admitting appointments to the event backbone.

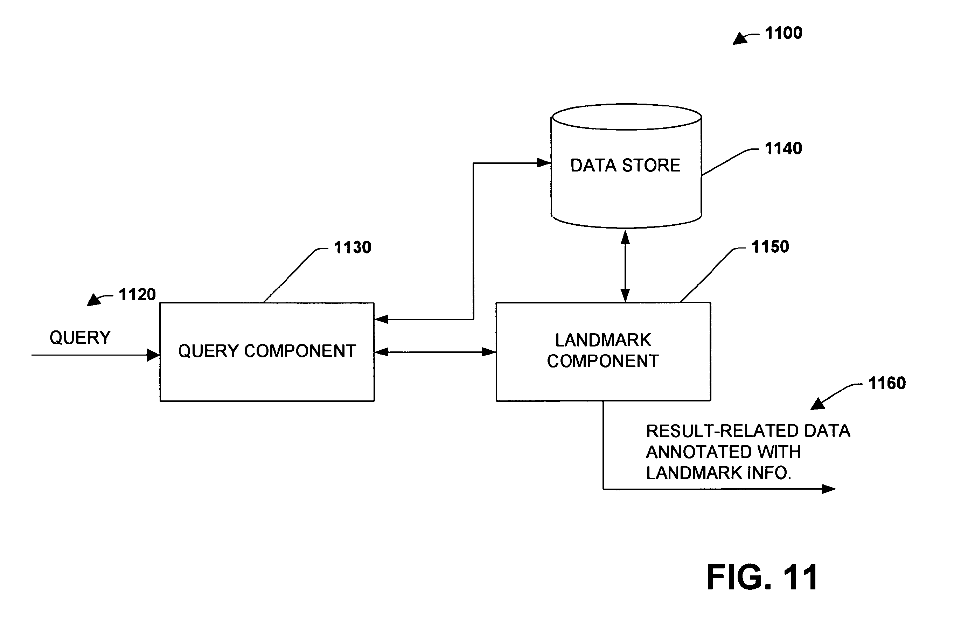

Fig. 11 illustrates a system 1100 in accordance with one particular aspect of the invention that facilitates computer-based searching for information. The system 1100 provides for design and analysis of timeline visualizations in connection with displaying results to queries based at least in part on an index of content. A query 1120 is received by a query component 1130 (which can be part of search engine that provides a unified index of information a user has been exposed to (*e.g*., including web pages, email, documents, pictures, audio...). The query component 1130 parses the query into portions relevant to effecting a meaningful search in accordance with the subject invention. The query component can access and populate a data store 1140 which may include information searched for. It is to be appreciated that the data store represents location(s) that store data. As such the data store 1140 can be representative of a distributed storage system, a plurality of disparate data stores, a single memory location, etc. A landmark component 1150 receives and/or accesses information from the query component 1130 as well as the data store, and anchors public (*e.g*., news, holidays) and/or personal (*e.g*., appointments, photos) landmark events to search results-related information. The landmark component 1150 outputs result-related data with landmark information at 1160. It is to be appreciated that the landmarks can be automatically generated and/or defined by a user. The system 1100 can index text and metadata of items (*e.g*., documents, visited web pages, and emails) that a user has been exposed to so as to provide a fast and easy manner to search over content. Thus, the system 1100 exploits value of extending a basic time view by adding public landmarks (*e.g*., holidays, important news events) and/or personal landmarks (*e.g*., photos, significant calendar events), wherein results of searches can be presented with an overview-plus-detail timeline visualization.

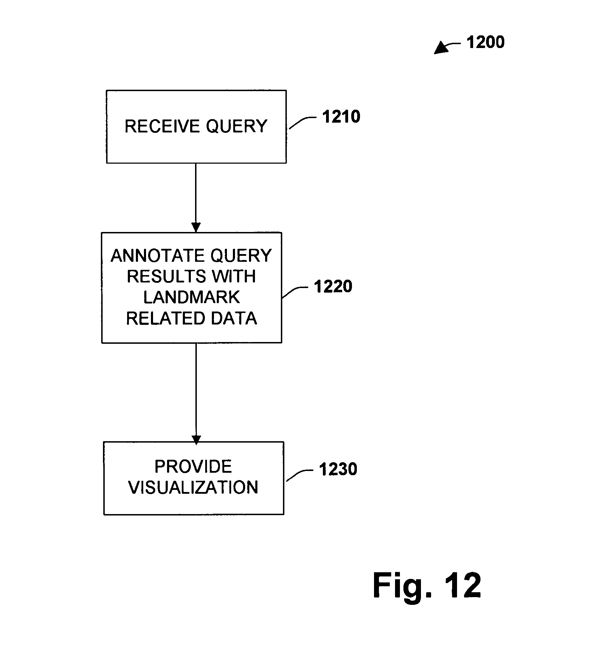

Fig. 12 illustrates a high-level methodology 1200 in accordance with one particular aspect of the subject invention. At 1210, a query is received. At 1220, query-related results data is anchored/annotated with landmark related data. At 1230, a timeline visualization is provided that displays results of the query based at least in part on an index of content.

The psychology literature, contains abundant discussion of *episodic memory,* the theory that memories about the past may be organized by *episodes,* which include information such as the location of an event, who was present, and what occurred before, during, and after the event. Research also suggests that people use routine or extraordinary events as "anchors" when trying to reconstruct memories of the past. Time of a particular event can be recalled by framing it in terms of other events, either historic or autobiographical. Visualization in connection with the subject invention harnesses these ideas by annotating a base timeline with personal and/or public landmarks when displaying the results of users' searches over personal content.

A study of memory for computing events showed that people forgot a significant number of computing tasks they had performed one month in the past. Their knowledge of a temporal order of those tasks had also decayed after one month, but when prompted by videos and photographs of their work during a target time period, they were able to recall significantly more of the tasks they had performed and were able to more accurately remember the actual sequence of those tasks. More generally, research on encoding specificity emphasizes interdependence between what is encoded and what cues are later successful for retrieval. Memory also depends on the reinstatement of not only item-specific contexts, but also more general learning contexts.

A large body of research on efficient searching exists, including work on visualizing search results in a matrix whose rows and columns could be ordered by a variety of user-specified parameters, work suggesting that textual and 2D interfaces are generally more efficient than 3D interfaces for most search tasks, and research on displaying categorical, summary, and/or thumbnail information with search results. The subject invention employs utility of timelines and temporal landmarks for guiding the search over content (*e.g*., personal content). Time is a common organizational structure for applications and data. Plaisant, *et al.'s* LifeLines (*See* Plaisant, C., Milash, B., Rose, A., Widoff, S., and Shneiderman, B. LifeLines: Visualizing Personal Histories. *Proceedings of CHI 1996,* 221-228) takes advantage of the time-based structure of human memory by displaying personal histories in a timeline format. Kumar, *et al*.'s work (*See* Kumar, V., Furuta, R., and Alien, R. Metadata Visualization for Digital Libraries:
Interactive Timeline Editing and Review. *Proceedings of the 3 rd ACM Conference on Digital Libraries* (1998), 126-133) on digital libraries uses timelines to visualize topics such as world history and stock prices, as well as metadata about documents in the library, such as publication date. Rekimoto's "time-machine computing" (*See* Rekimoto, J. Time-Machine Computing: A Time-centric Approach for the Information Environment. *Proceedings of UIST 1999*, 45-54) leverages the fact that people's activities are closely associated with times by allowing users to find old documents via "time-travel" to a prior version of their desktop where the target items were present. Fertig, *et al*.'s LifeStreams (*See* Rekimoto, J. Time-Machine Computing: A Time-centric Approach for the Information Environment. *Proceedings of UIST 1999*, 45-54) presents the user's personal file system in timeline format. "Forget-Me-Not" is a ubiquitous computing system that serves as a memory augmentation device by gathering information about daily events from other devices in the environment, and allowing perusal and filtering of those records. Meetings with coworkers (time, location, and names of people present), phone calls, and emails are examples of the type of data collected and available as memory cues. "Save Everything" (*See* Hull, J. and Hart, P. Toward Zero Effort Personal Document Management. *IEEE Computer* (March, 2001), 30-35) has a similar approach, collecting various data about documents and then allowing querying using personal metadata such as the manner of a document's acquisition (*e.g*., fax vs. email vs. photocopying) or the relevant activities occurring at the time of the data's acquisition. Minneman and Harrison's Timestreams (*See* Minneman, S. and Harrison, S. Space, Timestreams, and Architecture: Design in the Age of Digital Video. *Proceedings of the Third International International Federation of Information Processing WG 5.2 Workshop on Formal Design Methods for CAD* (1997)) use everyday activities (*e.g*., speaking, drawing sketches, typing notes) to index into audio and video streams. In contrast to these efforts, the system 1100 in accordance with the subject invention uses a variety of personal and public landmarks as memory cues to explore whether such context provides useful memory prompts for efficiently searching personal content. While previous research efforts have individually explored timeline-based visualizations, contextual cues for retrieval, or other methods for increasing search efficiency, the subject invention bridges all three areas by using the metaphor of a timeline combined with contextual cues in searching over content (*e.g*., personal content).

### VISUALIZATION

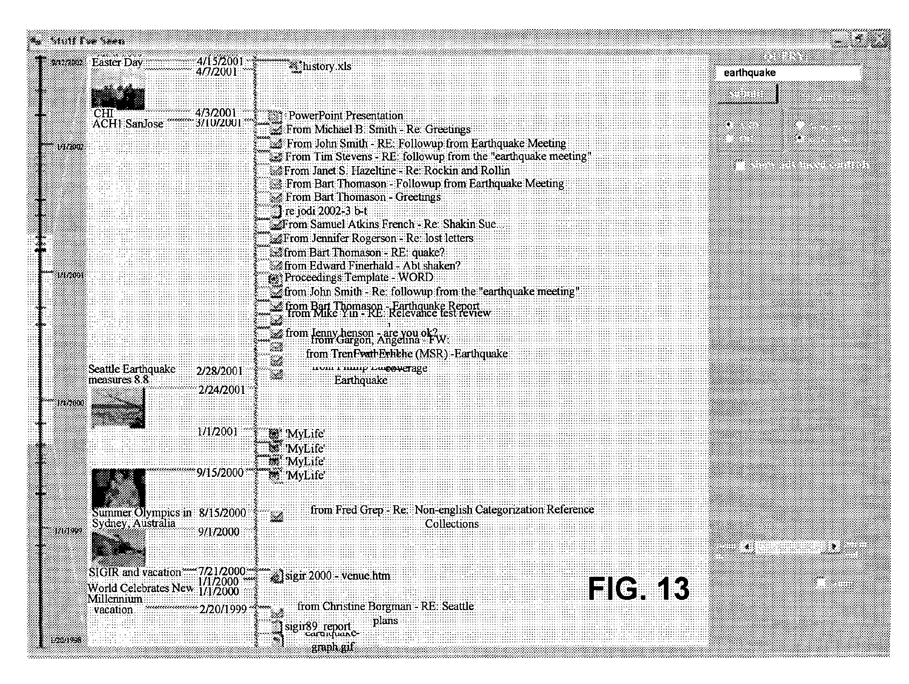

Fig. 13 is an exemplary screenshot representation of a timeline visualization in accordance with the subject invention. An overview area at the left shows a timeline with hash marks representing distribution of search results over time. A highlighted region of the overview timeline corresponds to a segment of time displayed in a detailed view. To the left of the detailed timeline backbone, basic dates as well as landmarks drawn from news headlines, holidays, calendar appointments, and digital photographs provide context. To the right of the backbone, details of individual search results (represented by icons and titles) are presented chronologically.

To test the value of annotating timelines with temporal landmarks, a prototype was developed that provides an interactive visualization of results output by a search application. The visualization, displayed in Figure 13, has two main components for providing both overview and detail about the search results. The left edge of the display shows the overview timeline, whose endpoints are labeled as the dates of the first and last search result returned. Annual boundaries are also marked on the overview if the search results span more than one year, for example. Time flows from the top to the bottom of the display, with the most recent results at the top. The overview provides users with a general impression of the number of search results and their distribution over time. A portion of the overview is highlighted; this corresponds to the section that is currently in focus in the detailed area of the visualization. Users can interact with the overview timeline as if it were a scroll bar, by selecting the highlighted region (*e.g*., with a mouse cursor) and moving it to a different section of the timeline, thus changing the portion of time that is displayed in the detailed view. The detailed portion of the visualization shows a zoomed-in section of the timeline, corresponding to the slice of time highlighted in the overview area. Each search result is shown at the time when the document was most recently saved. An icon indicating the type of document (html, email, word processor, etc.) is displayed, as well as the title of the document (or subject line and author, in the case of email). By hovering the cursor over a particular search result, users can view a popup summary containing more detailed information about the object, including the full path, a preview of the first 512 characters of the document (or other amount), as well as *to-, from-,* and *cc*- information in the case of mail messages. Clicking on a result opens the target item with the appropriate application. Search results are displayed to the right of the backbone of the detailed timeline. The left-hand side of the backbone is used to present date and landmark information. Dates appear nearest the backbone. The granularity of dates viewed (hours, days, months, or years) depends upon the current level of zoom. Four types of landmarks may be displayed to the left of the dates: holidays, news headlines, calendar appointments, and digital photographs (can include more or less types). Each of the landmarks appears in a different color (can be similar colors). It is to be appreciated that the scale, ordering and placement of the aforementioned aspects can be suitably tailored in accordance respective needs.

### Public Landmarks

Public landmarks are drawn from incidents that a broad base of users would typically be aware of. Landmarks are given a priority ranking, and typically only landmarks that meet a threshold priority are displayed. For a prototype in accordance with the subject invention, all users saw the same public landmarks, although it is to be appreciated that different aspects of the invention can explore letting users customize their public landmarks adding, for instance, religious holidays that are important to them, or lowering the ranking of news headlines that they don't deem memorable.

### Holidays

A list of secular holidays commonly celebrated in the United States was obtained, and the dates those holidays occurred from 1994 through 2004, by extracting that information from a calendar. Priorities were manually assigned to each holiday, based on knowledge of American culture (e.g., Groundhog Day was given a low priority, while Thanksgiving Day was given a high priority). Holidays and priorities could easily be adapted for any culture.

### News Headlines

News headlines from 1994 - 2001 were extracted from the world history timeline that comes with a commercially available multimedia encyclopedia program. Because 2002 events were not available, inventors of the subject invention used their own recollections of current events to supply major news headlines from that year. Ten employees from an organization (none of whom were participants in a later user study) rated a set of news headlines on a scale of 1 to 10 based on how memorable they found those events. The averages of these scores were used to assign priorities to the news landmarks.

### Personal Landmarks

Personal landmarks are unique for each user. For the prototype, all of these landmarks were automatically generated, but for other aspects of the subject invention it is appreciated that users can have the option of specifying their own landmarks.

### Calendar Appointments

Dates, times, and titles of appointments stored in the user's calendar were automatically extracted for use as landmark events. Appointments were assigned a priority according to a set of heuristics. If an appointment was recurring, its priority was lowered, because it seemed less likely to stand out as memorable. An appointment's priority increased proportionally with the duration of the event, as longer events (for example such as conferences or vacations) seemed likely to be particularly memorable. For similar reasons, appointments designated as "out of office" times received a boost in score. Being flagged as a "tentative" appointment lowered priority, while being explicitly tagged as "important" increased priority.

### Digital Photographs

The prototype crawled the users' digital photographs (if they had any). The first photo taken on a given day was selected as a landmark for that day, and a thumbnail (64 pixels along the longer side) was created. Photos that were the first in a given year were given higher priorities than those which were the first in a month, which in turn were ranked more highly than those which were first on a day. Thus, as the zoom level changed an appropriate number of photo landmarks could be shown. The inventors did not explore more sophisticated algorithms for selecting photos to display, but it is to be appreciated that such techniques (*See* Graham, A., Garcia-Molina, H., Paepcke, A., and Winograd, T. Time as Essence for Photo Browsing Through Personal Digital Libraries. *Proceedings of the Second ACM*/*IEEE-CS Joint Conference on Digital Libraries* (2002), 326-335, or by Platt, J. AutoAlbum: Clustering Digital Photographs Using Probabilistic Model Merging. *IEEE Workshop on Content-Based Access of Image and Video Libraries 2000,* 96-100) are contemplated with respect to the subject invention and are intended to fall within the scope of the hereto appended claims.

### STUDY

To evaluate concepts behind the prototype, a user study was conducted. Goals were to learn whether a timeline-based presentation of search results was helpful to users, and whether different types of landmarks improved the utility of the timeline view for searching. Both quantitative and qualitative data were gathered to investigate those issues.

### Participants

The subjects were twelve employees from an organization, all of whom were men aged twenty-five to sixty. A prerequisite for participation in the study was being a user of a search system (e.g., Stuff I've Seen (SIS)).

### Preparation

The day before each subject came to a usability lab, they were asked to do two things. First, the inventors asked subjects to install a program that extracted the titles of all of their non-private appointments from their calendar, and then e-mail that list of titles to the inventors. This information was employed to create from two to eight personalized queries for each participant, based on educated guesses about their appointments (*e.g*., if they had an appointment called "trip to Florida" the inventors might prepare a question like "Find the webpage you used when buying your airline tickets to Florida", or if they had an appointment called "CHI 2002" the inventors might ask them to find the paper they had submitted to CHI 2002).

Second, each subject was sent *a .pst* file (*e.g.,* a repository of Microsoft Outlook^{TM} email messages) so that the SIS application running on their machine would have time to index the contents of that file before they arrived for the study. This file contained a collection of messages that had been sent to a large number of people in the organization (*e.g*., announcements of talks, holiday parties, promotions, etc.), which everyone would have received at some point. Although the inventors knew everyone had received these messages since they were originally sent to large mailing lists, the inventors did not know in advance whether individual participants archived such mail or deleted it, so the inventors sent them the .pst file in order to facilitate that the target items were in their index.

### Method

When participants came to the usability lab, they were asked to use Windows XP's Remote Desktop feature to access their office computer. While the participant toured the lab, the inventors installed a visualization client in accordance with the subject invention on their machine. Participants first filled out a questionnaire asking for demographic information as well as information about their searching and filing habits and about ways they remembered information. Next they read a tutorial and performed two practice searches using the timeline interface. They were given as much time as they needed to complete the tutorial and were allowed to ask questions. The experiment began after the tutorial was completed.

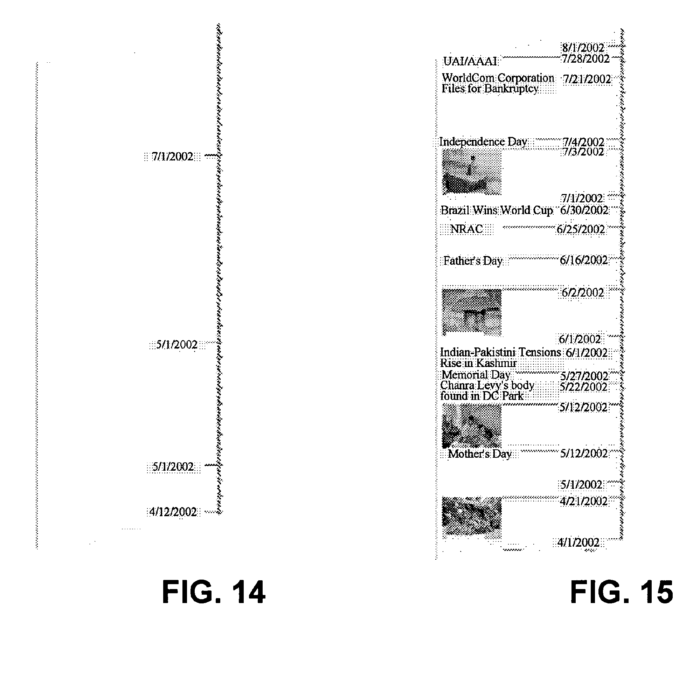

The experiment had a within-subjects design. Each participant was given a series of tasks to complete using two different interfaces. For half of the tasks, they saw their search results presented in the context of a timeline annotated only by dates (Fig. 14), and for the other half they saw the timeline annotated by calendar appointments, news headlines, holidays, and digital photos (if they had any stored on their computer) in addition to the basic dates (Fig. 15). The conditions were counter-balanced to avoid learning effects, so that half of the participants experienced the landmark condition before the dates-only condition, and the other half experienced the conditions in the reverse order. To avoid ordering effects, the order of questions was randomly changed for every pair of participants.

The inventors used two kinds of questions: thirty questions common to all participants, and 2-8 unique personalized questions. The first fifteen questions in each of the two conditions involved finding items which the inventors knew had been sent to a large number of employees, and which the inventors had included in the .pst file the inventors had installed the previous day. For each of these thirty common tasks, the inventors provided participants with a pre-determined query to issue, and instructed them not to change this query. The inventors chose to use pre-set queries because their goal was to test how well the timeline and landmarks helped users to navigate among their search results, and the inventors did not want to inadvertently end up testing how well the user was able to formulate a query. Thus, the inventors chose queries that would ensure that the target item would appear somewhere on the timeline, but that were broad enough that many other results would also appear.

At the end of each set of common questions, the inventors asked a few questions that the inventors had customized for each user based on the subject lines from their calendar appointments that the inventors had extracted the day before. Although these questions were different for each participant, the inventors felt they were important to add because they targeted more personal and memorable documents than the company-wide email messages. For these personal tasks, users were allowed to enter a query of their choosing and to reformulate the query to refine their search if they desired.

Once a query had been issued, users could navigate the timeline and inspect the search results by looking at the icons and titles, hovering for popup summaries with more detailed information, or clicking to open the actual document. When they had found the target item, they clicked a large button marked "Found It," and were automatically presented with the next task and query. If they were unable to locate the target item, there was also a button marked "Give Up," which allowed them to proceed to the next question. During the experiment, software logged all the details of their interaction, including the number of search results returned for each query, the number of landmarks of various types that were displayed, and information on the users' hovering, clicking, and overall timing of interactions.

After completing all of the tasks, subjects filled out another questionnaire asking for feedback about the usability of the software, the utility of the timeline presentation and the various types of landmarks, and for free-form comments.

In summary, each of the 12 study participants were exposed to both of the experimental conditions―using the timeline with dates and landmarks, and using the timeline with dates only. In each condition, participants used the visualization to answer two types of questions―fixed questions about email that had been sent to large distribution lists and personalized questions custom-picked for each subject.

### Results

### Search Time

Analysis was performed on the median search times for each participant to help mitigate common skewing of human performance times. Here the inventors only looked at questions common to all participants, to insure a fair comparison. A paired-sample t-test of the median search times for each participant indicated that times for the Landmark condition were significantly faster than the date-only condition, t(11)=2.33, p<0.05. A comparison of the average of median search times is shown in Figure 14 (±standard error about the mean). For the landmark condition, the average of the median search times was 18.37 seconds, while for the dates-only condition this value was 24.25 seconds. Unsurprisingly, timing data for personalized questions were extremely noisy; and there was no significant difference between the two conditions for those queries

### Questionnaire

In addition to the timing data, participants completed questionnaires at the beginning and conclusion of the experiment. Participants first entered some demographic information followed by a number of questions using a 7- point Likert scale. (A score of 1= "Strongly Disagree" and 7 = "Strongly Agree." *E.g*., "I liked using this software" or "When I need to find old documents or email, it is relatively easy to do so."). Finally, participants answered a number of free-form questions (*e.g*., "Are there certain types of search tasks for which you think landmarks would help you search more efficiently?").

At the start of each session, before seeing the visualization, subjects answered a series of questions about their current strategies for locating documents (Table 1). The three most highly rated attributes for searching were topic, people and time. Existing search tools support access by topic and people, but provide less support for time-oriented search. The visualization helps remedy this by allowing a keyword-based search to generate an initial set of results, coupled with a rich time display for navigation among results.

Before beginning the study session, subjects were also asked to rate the importance of different types of landmarks for recalling events (Table 2). It is interesting to note that public events (world events and holidays) received lower ratings than more personalized events. One user commented, *"Photos could easily be useful, as are calendar appts. But news events and holidays are less important. I mean, I know Halloween is in October... and Xmas is in December. Calling that out doesn't add information."* Another user said, "*For me it's more events in my life, then world wide events. Of course 9*/*11 is a big thing, but for me I think of what happened before I went to Africa, or after I moved into the new house, etc*."

An interesting avenue for future work would be to extend the study of the date-only versus all-landmarks conditions by distinguishing between different types of events― running "personal landmarks" and "public landmarks" conditions in addition to the two conditions explored here. After finishing the experiment, participants evaluated the general usefulness of the timeline interface (Table 3). Participants generally found the time-based presentation of results useful, although it would be worthwhile to explore further whether certain classes of search tasks are better suited to time-based presentation of results and other types of tasks might work best with alternate organizational schemes. One participant suggested the landmarks were most useful when *"looking for time- or event-related mail: finding Rick's mail about airport closures is pretty coupled to Sept. 11.*"

Although the vertical presentation of the timeline was well received, many users wanted the option of reversing the flow of time such that more recent search results were displayed near the bottom of the screen. This preference about the direction of time was often related to whether their email client displayed newer messages at the top or bottom of the message queue. As can be appreciated, the present invention can employ various timeline renderings (*e.g*., horizontal timelines, reverse direction timelines).

Users generally found the overview provided in the visualization to be useful (one user commented, *"I liked the way the little horizontal lines showed bursts of activity. That way I could figure out what time period stuff happened.*"), but many users found it confusing to navigate through the search results by selecting a section of the overview timeline (another user said, *"Adjusting the time scale on the Overview pane didn't seem intuitive to me*").

### CONCLUSIONS

The inventors developed and evaluated a timeline-based visualization of search results over personal content. Results on episodic memory inspired them to augment the timeline with public (news headlines and holidays) and personal (calendar appointments and digital photographs) landmark events, in hopes that this added context would aid people in locating the target of their search. A user study found that there was a statistically significant time savings for searching with the landmark-augmented timeline compared to a timeline marked only by dates. Additionally, the inventors gathered important feedback about the way users believe that they remember events and about their reactions to the visualization. This work demonstrates the utility of adding global and personal context to the presentation of search results, as well as suggesting directions for future study.

In view of at least the above, the inventors contemplate relative value of different kinds of temporal landmarks in reviewing search results, and for investigating, more generally, when timeline-centric views are most useful for finding target results of interest. It is likely, for example, that the distribution of items over time returned for a particular query will influence the overall utility of a timeline view for finding items. There are a number of other opportunities for refining the system. Users reported some difficulty in navigating the timeline and the inventors would like to improve the control of navigation via better coupling of zooming and translation in time. Accordingly, one particular aspect of the subject invention can refine heuristics (or other models) for selecting and ranking landmarks (from all sources), and in exploring different types of summary landmarks. For example, shading segments of the overview timeline with different colors to indicate years or seasons within a year can be employed. Landmarks related to the search results themselves could also be identified, such as key attributes about the content and structure of documents. In addition to passively displaying landmarks, users can combine landmarks and more traditional search terms in formulation of a query, enabling users to search "by landmark", *e.g.*, saying something like "show me all documents that I composed right before the project review with my manager" or "show me all emails I received the week of the earthquake."

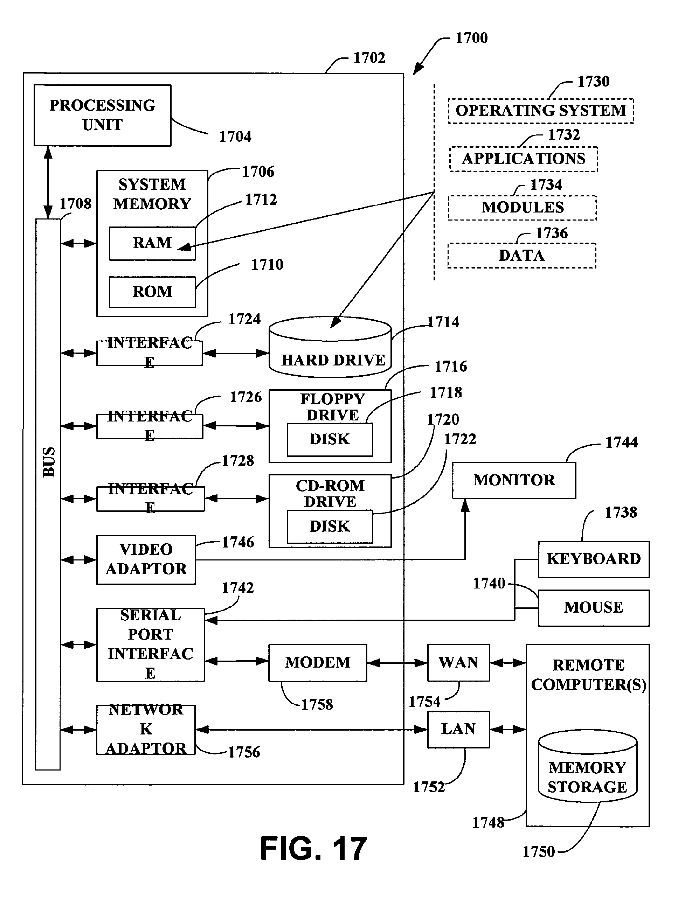

With reference to Fig. 17, an exemplary environment 1700 for implementing various aspects of the invention includes a computer 1702, the computer 1702 including a processing unit 1704, a system memory 1706 and a system bus 1708. The system bus 1708 couples system components including, but not limited to the system memory 1706 to the processing unit 1704. The processing unit 1704 may be any of various commercially available processors. Dual microprocessors and other multi-processor architectures also can be employed as the processing unit 1704.

The system bus 1708 can be any of several types of bus structure including a memory bus or memory controller, a peripheral bus and a local bus using any of a variety of commercially available bus architectures. The system memory 1706 includes read only memory (ROM) 1710 and random access memory (RAM) 1712. A basic input/output system (BIOS), containing the basic routines that help to transfer information between elements within the computer 1702, such as during start-up, is stored in the ROM 1710.

The computer 1702 further includes a hard disk drive 1714, a magnetic disk drive 1716, (*e.g*., to read from or write to a removable disk 1718) and an optical disk drive 1720, (*e.g*., reading a CD-ROM disk 1722 or to read from or write to other optical media). The hard disk drive 1714, magnetic disk drive 1716 and optical disk drive 1720 can be connected to the system bus 1708 by a hard disk drive interface 1724, a magnetic disk drive interface 1726 and an optical drive interface 1728, respectively. The drives and their associated computer-readable media provide nonvolatile storage of data, data structures, computer-executable instructions, and so forth. For the computer 1702, the drives and media accommodate the storage of broadcast programming in a suitable digital format. Although the description of computer-readable media above refers to a hard disk, a removable magnetic disk and a CD, it should be appreciated by those skilled in the art that other types of media which are readable by a computer, such as zip drives, magnetic cassettes, flash memory cards, digital video disks, cartridges, and the like, may also be used in the exemplary operating environment, and further that any such media may contain computer-executable instructions for performing the methods of the present invention.

A number of program modules can be stored in the drives and RAM 1712, including an operating system 1730, one or more application programs 1732, other program modules 1734 and program data 1736. It is appreciated that the present invention can be implemented with various commercially available operating systems or combinations of operating systems.

A user can enter commands and information into the computer 1702 through a keyboard 1738 and a pointing device, such as a mouse 1740. Other input devices (not shown) may include a microphone, an IR remote control, a joystick, a game pad, a satellite dish, a scanner, or the like. These and other input devices are often connected to the processing unit 1704 through a serial port interface 1742 that is coupled to the system bus 1708, but may be connected by other interfaces, such as a parallel port, a game port, a universal serial bus ("USB"), an IR interface, etc. A monitor 1744 or other type of display device is also connected to the system bus 1708 via an interface, such as a video adapter 1746. In addition to the monitor 1744, a computer typically includes other peripheral output devices (not shown), such as speakers, printers etc.

The computer 1702 may operate in a networked environment using logical connections to one or more remote computers, such as a remote computer(s) 1748. The remote computer(s) 1748 may be a workstation, a server computer, a router, a personal computer, portable computer, microprocessor-based entertainment appliance, a peer device or other common network node, and typically includes many or all of the elements described relative to the computer 1702, although, for purposes of brevity, only a memory storage device 1750 is illustrated. The logical connections depicted include a LAN 1752 and a WAN 1754. Such networking environments are commonplace in offices, enterprise-wide computer networks, intranets and the Internet.

When used in a LAN networking environment, the computer 1702 is connected to the local network 1752 through a network interface or adapter 1756. When used in a WAN networking environment, the computer 1702 typically includes a modem 1758, or is connected to a communications server on the LAN, or has other means for establishing communications over the WAN 1754, such as the Internet. The modem 1758, which may be internal or external, is connected to the system bus 1708 via the serial port interface 1742. In a networked environment, program modules depicted relative to the computer 1702, or portions thereof, may be stored in the remote memory storage device 1750. It will be appreciated that the network connections shown are exemplary and other means of establishing a communications link between the computers may be used.

In accordance with one aspect of the present invention, the filter architecture adapts to the degree of filtering desired by the particular user of the system on which the filtering is employed. It can be appreciated, however, that this "adaptive" aspect can be extended from the local user system environment back to the manufacturing process of the system vendor where the degree of filtering for a particular class of users can be selected for implementation in systems produced for sale at the factory. For example, if a purchaser decides that a first batch of purchased systems are to be provided for users that do should not require access to any junk mail, the default setting at the factory for this batch of systems can be set high, whereas a second batch of systems for a second class of users can be configured for a lower setting to all more junk mail for review. In either scenario, the adaptive nature of the present invention can be enabled locally to allow the individual users of any class of users to then adjust the degree of filtering, or if disabled, prevented from altering the default setting at all. It is also appreciated that a network administrator who exercises comparable access rights to configure one or many systems suitably configured with the disclosed filter architecture, can also implement such class configurations locally.

What has been described above includes examples of the present invention. It is, of course, not possible to describe every conceivable combination of components or methodologies for purposes of describing the present invention, but one of ordinary skill in the art may recognize that many further combinations and permutations of the present invention are possible. Accordingly, the present invention is intended to embrace all such alterations, modifications and variations that fall within the spirit and scope of the appended claims. Furthermore, to the extent that the term "includes" is used in either the detailed description or the claims, such term is intended to be inclusive in a manner similar to the term "comprising" as "comprising" is interpreted when employed as a transitional word in a claim.

## Claims

1. A system that facilitates computer-based searching, comprising:
a query component that receives information related to a search for information; and
a landmark component that employs content-based landmark information to facilitate the search for information, the landmark information corresponding to contextual information related to event(s) memorable to an originator of the search.

2. The system of claim 1 providing timeline visualizations in connection with displaying results to the search based at least in part on an index of personal content.

3. The system of claim further comprising a search engine that provides a unified index of information to which a user has been exposed.

4. The system of claim3, the information comprising at least one of: web pages, email, documents, pictures, and audio.

5. The system of claim 2, results of searches are presented with an overview-plus-detail timeline visualization.

6. The system of claim 5, further providing a summary view that shows distribution of search hits over time.

7. The system of claim 5, further providing a detailed view that allows for inspection of individual search results.

8. The system of claim 7, annotating returned items with icons and/or short descriptions.

9. The system of claim 1, the landmark component extending a basic time view by adding public landmarks and/or personal landmarks.

10. The system of claim 1, employing contextual information to support searching through content.

11. The system of claim 1, anchoring timeline-based presentations of search with public and/or personal landmark events.

12. The system of claim 1, further comprising an indexing component that can index text and/or metadata of items that a user has been exposed to so as to facilitate a fast and easy manner to search over content.

13. A computer readable medium having stored thereon the components of claim 1.

14. A method that facilitates computer-based searching, comprising:
receiving information related to a search for information;
employing content-based landmark information to facilitate the search for information, the landmark information corresponding to contextual information related to event(s) memorable to an originator of the search; and
providing a timeline visualization of search results based at least in part upon an index of a subset of the contextual information.

15. The method of claim 14 further comprising employing one or memorability models to determine the landmark information.

16. The method of claim 15, the memorability models include at least one of a voting model, a heuristic model, a rules model, a statistical model, an inference model, and a complimentary model.

17. The method of claim 16, the complimentary model is based upon patterns of forgetfulness.

18. The method of claim 14 further comprising employing the landmark information in a browser interface that associates one or more events relating to the landmark information to one or more items that are retrievable by the browser.

19. A system that facilitates computer-based searching, comprising:
means for receiving information related to a search for information;
means for employing content-based landmark information to facilitate the search for information, the landmark information corresponding to contextual information related to event(s) memorable to an originator of the search; and
means for providing a timeline visualization of search results based at least in part upon an index of a subset of the contextual information.

20. A system employing memorability models, comprising:
one or more memorability models that automatically capture an ability of people to recognize events as landmarks in time; and
an application that employs the memorability models to facilitate processing of information in accordance with the events.

21. The system of claim 20, the memorability models include procedures and policies for assigning a measure of memorability to events that can be employed by various computer-based applications to aid users in processing, receiving, and/or communicating information.

22. The system of claim 21, the events can include at least one of appointments, annotations in a user's calendar, holidays, news stories over time, and images.

23. The system of claim 20, the memorability models are employed to provide a personalized index containing landmarks in time, the index is employed in at least one application relating to browsing directories of information and in reviewing results of a search engine.

24. The system of claim 20, the memorability models can include at least one of voting models, heuristic models, rules models, statistical models, and complimentary models that are based on patterns.

25. The system of claim 24, the voting models automatically poll a set of users in order to score the memorability of public events.

26. The system of claim 25, the score is based on scalar measures of memorability that include at least one of salience of news stories taken from a corpus of news stories and querying a set of people to assign a value.

27. The system of claim 24, the heuristic models utilize properties of messages and create informal policies that assign scores or deterministic categories of memorability based on functions of the properties.

28. The system of claim 27, further comprising a heuristic function that analyzes the increasing duration of events on a calendar as positively influencing the memorability of the events.

29. The system of claim 28, the heuristic function is applied to which images or subsets of images from a set of images serve as the most memorable of sets of images taken at the event based one or more properties of the images.

30. The system of claim 29, the properties include at least one of a composition of objects in a scene, a color histogram, faces recognized, features involving the sequence and temporal relationships among pictures, a picture associated with short inter-picture intervals, a capturing of excitement of a photographer about an aspect of the events, and properties that indicate that a user's activity with regard to the image.

31. The system of claim 30, the user's activity includes examining or displaying the image with longer or shorter dwell time, editing the image, cropping the image, and renaming the image.

32. The system of claim 30, further comprising automated analysis of image quality including focus and orientation.

33. The system of claim 24, the rules models include rules for automatically assigning measures of memorability to news stories that include properties relating to at least one of the number of news stories, persistence in the media, number of casualties, the dollar value of the loss associated with the news story, features capturing dimensions of surprise or atypical, and the proximity to the user of the event.

34. The system of claim 33, the statistical models employ machine learning methods that provide models which predict the memorability of items, the statistical models include the use of Bayesian learning, which can generate at least one of Bayesian dependency models (such as Bayesian networks), naïve Bayesian classifiers, and Support Vector Machines (SVMs).

35. The system of claim 24, further comprising a trainer component that takes explicit examples of landmark items or items that are forgotten.

36. The system of claim 35, the trainer is supplied with examples identified through implicit training.

37. The system of claim 24, the complimentary models describe the use of variants of memorability which are focused on inferring the likelihood that users will not recall a forthcoming event.

38. The system of claim 37, the complimentary models utilize inferences in applications to highlight in a selective manner the information that a user is likely to forget in a visually salient manner, or to change the timing or alerting of information in accordance with the likelihood that the information will not be remembered.

39. The system of claim 37, the complimentary models are combined with messaging and reminding systems including context-sensitive costs and benefits of transmitting information and alerting a user about information that is possibly forgotten.

40. The system of claim 20, further comprising a threshold adjustment allowing landmark events from a user's calendar to be displayed that have a higher likelihood than a threshold of being memorable, per the setting of the adjustment.

41. The system of claim 40, further comprising a display that progressively lightens events with progressively lower likelihoods of being a landmark.

42. The system of claim 41, further comprising a step that assigns intensity as a function of membership of an event within different ranges of likelihood of being a landmark.

43. The system of claim 20, further comprising a training interface that fetches a file of a user's calendar appointments over the years and allows the user to indicate whether appointments serve as memory landmarks.

44. The system of claim 43, the training interface further comprises a train button that creates a statistical classifier that takes multiple properties of events on a user's calendar and predicts the likelihood that each event is a landmark event.

45. The system of claim 44, the likelihood is based on the following expression:
p(memory landmark | E1...En), wherein p is a probability and E1... En is evidence relating to one or more event properties.

46. The system of claim 20, further comprising an inference model to process memorability variables including at least one of, whether or not peers are at a meeting, the day of week, the time of day, the duration of a meeting, whether the meeting is recurrent, the time set for early reminding about a meeting, the role of a user, did the meeting come via an alias or from a person, how many attendees are at the meeting, are a user's direct reports, manager, or manager's manager at the meeting, who is the organizer of the meeting, the subject of the meeting, the location of the meeting, and how did the user respond to the meeting request.

47. The system of claim 46, further comprising processing at least one of "organizer atypia," "location atypia," and "attendees atypia" that are computed from a user's appointment store and capture the rarity or "atypia" of properties of an event or appointment.

48. The system of claim 47, further comprising discretizing typicality for a Location, an Organizer, and an Attendee into states based on ranges of frequency.

49. The system of claim 20, further comprising one or more controls that are selected by users for controlling how and when events are displayed.

50. A method for applying memorability information, comprising:
automatically labeling events or items with numerical or categorical labels according to a measure of the likelihood that an item will be recalled, recognized as a landmark, or be most representative of an event or time; and
applying the labeling to information-management applications.

51. The method of claim 50, further comprising employing mathematical functions that assign a scalar measure of salience of events or items as being recalled, recognized as landmarks, or most representative of events or times.

52. The method of claim 51, further comprising at least one of:
applying statistical models of memorability via machine learning methods that are trained implicitly or with an explicit training system;
collecting information about a sample of memorable or non-memorable events or items that provides real-time inference or classification about the likelihood that an event or items as being recalled, recognized as landmarks, or be most representative of events or times; and
providing a probability distribution over different degrees of the event or item.

53. The method of claim 50, further comprising automatically filtering a stream of heterogeneous events and content, so as to selectively store events for log of lifetime events.

54. The method of claim 50, further comprising hierarchically browsing a log of heterogeneous events and content or browsing data at different levels of temporal precision.

55. The method of claim 50, further comprising employing representative landmarks and memorability to selectively choose pictures for an ambient display of pictures drawn from a picture library.

56. The method of claim 50, further comprising employing representative memory landmarks and memorability to selectively choose a set of pictures in a slide show over time or at different points in time about one or more events, under constraints in the total number of slides that a user desires to show.

57. The method of claim 50, further comprising employing representative memory landmarks and memorability to selectively choose a set of items to characterize or summarize the contents of a corpus of items.

58. The method of claim 57, the items include at least one of an image, a photo library, a thumbnails of graphics or photo images displayed on files, items, or folders of documents.

59. The method of claim 50, the information-management applications are applied to at least one of a memorability application, relating to will an item be recalled and understood, a memorable landmark relating to will an item be viewed as a milestone in time, and a representative landmark relating to is the item representative of a period of time, event, or sequence of events.

60. A method for determining reminders, comprising:
automatically training models from data; and
performing inference about items that are potentially forgotten.

61. The method of claim 60, further comprising:
inferring a likelihood that an item will be forgotten; and
performing a cost-benefit analysis of an expected value of reminding a user about the item.

62. The method of claim 60, further comprising performing expected-utility decision making about if and when to come forward to remind a user about something that they are likely to forget given an item type and context in view of a cost of an interruption.

63. The method of claim 60, further comprising controlling of alerting about reminders in desktop applications or mobile devices via the incorporation of the disruptiveness and the cost of a transmission.

64. The method of claim 60, further comprising automatically assisting patients with various cognitive deficits that may lead to memory aberrancies.

65. The method of claim 64, further comprising automatically predicting the likelihood that a patient with Alzheimer's disease is at a particular stage of the illness.

66. The method of claim 65, further comprising at least one of automatically providing audiovisual cues to users and automatically providing ideal reminders.
